(19) Europäisches Patentamt / European Patent Office / Office européen des brevets

(11) **EP 2 336 740 B1**

(12) **EUROPEAN PATENT SPECIFICATION**

(45) Date of publication and mention
of the grant of the patent:
**12.02.2014 Bulletin 2014/07**

(51) Int Cl.:
*G01J 5/00* *(2006.01)*    *G01N 25/72* *(2006.01)*
*G01N 33/38* *(2006.01)*    *C03B 9/41* *(2006.01)*
*G01J 5/02* *(2006.01)*    *G01J 5/08* *(2006.01)*
*G01J 5/10* *(2006.01)*

(21) Application number: **09075545.5**

(22) Date of filing: **10.12.2009**

(54) **Method and system for monitoring a glass container forming process**

Verfahren und System zur Überwachung von Prozessen zum Formen von Glasbehältern

Procédé et système de surveillance de procédé de formation de récipient en verre

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(43) Date of publication of application:
**22.06.2011 Bulletin 2011/25**

(73) Proprietor: **Emhart Glass S.A.**
**6330 Cham (CH)**

(72) Inventors:
 • **Holtkamp, Mark Edwin**
 **9721 AM Groningen (NL)**
 • **Brummelman, Teunis René**
 **9617 EC Harkstede (NL)**

(74) Representative: **Warren, Anthony Robert**
**Baron Warren Redfern**
**Cambridge House**
**100 Cambridge Grove**
**Hammersmith**
**London**
**W6 0LE (GB)**

(56) References cited:
**WO-A1-2004/011935**    **US-A- 4 492 476**
**US-A- 5 032 727**    **US-A1- 2007 102 628**

• DR JOHN CHAN: "Automated inspection and
container monitoring at the hot end"
INTERNATIONAL GLASS REVIEW, CONTRACT
COMMUNICATIONS, LONDON, GB, 1 January
1997 (1997-01-01), pages 109-111, XP002991197
ISSN: 1359-4974
• MICHELETTI R: "AUTOMATIC VISUAL
INSPECTION FOR GLASS PRODUCTION"
ISMCR. PROCEEDINGS OF THE
INTERNATIONAL SYMPOSIUM ON
MEASUREMENTAND CONTROL IN ROBOTICS,
XX, XX, 1 January 1998 (1998-01-01), pages
127-131, XP009069952

**Description**

The present invention relates to a method and system for monitoring a glass container forming process. The forming process is accomplished by a forming machine which may contain multiple independent sections, each section consisting of at least one forming station. The method comprises the steps of measuring radiation emitted by each hot glass container immediately after the forming machine. Based on these measurements, information and control signals may be generated to adjust the glass container forming process in order to improve the quality of the glass containers and by doing so reduce the number of bad glass containers produced.

An article by Dr. John Chan entitled "Automated inspection and container monitoring at the hot end" in international Glass Review, Contract Communications, London, GB., 1st January 1997, pages 109-111, and related US-A-5,583,337, propose a system of the above type in which the system's operation is synchronised to that of the I.S. machine. This allows individual mold cavities to be tracked and monitored for performance, i.e. the hot glass containers are monitored on a per cavity basis.

In the system proposed in our US-A-2007/0102628, the hot glass bottles are, likewise also monitored on a per cavity basis. A vertical strip of data is defined and stored, representing the averaged data for a particular bottle from a particular mold cavity. A computer screen displays a plurality of adjacent vertical data strips derived from bottles from the same mold cavity, referred to as the mold/time picture for a particular mold, which allows an operator to determine whether the bottle forming process associated with that particular mold is stable or unstable. The computer screen, for bottles made in a twelve section quad gob machine, could present 48 of these mold/time pictures, one for each of the 48 molds.

Reference is also made to an article by Roberto Micheletti entitled "Automatic visual inspection for glass production" in the ISMCR. Proceedings of the International Symposium on Measurement and Control in Robotics, 1st January 1998, pages 127-131. Unlike the systems discussed above, the Micheletti system is not concerned with measuring radiation from hot glass bottles, but with a system based on computer vision for detecting flaws or cracks in cold bottles. The system utilises the image comparison approach that involves template matching techniques where the pattern under test (P.U.T.) is compared with a reference pattern (R.P.). Data analysis involving normalisation uses tonal measures of mean and deviation of the gray levels, and determines the dissociation factor between the P.U.T. and R.P. The tonal measures used for quality control of glass are the average image intensity of each row of the picture and the deviation of the average image intensity of each row from the general mean.

A system of the type discussed in the opening paragraph is also disclosed in WO 2004/011935 corresponding to European Patent EP 1525469 B1, which describes a system for analysing and monitoring a production process for glass products. The system is sensitive to radiation in the Near Infra Red (NIR) region solely and it measures the NIR radiation of hot glass products, determines the average radiation intensity for at least two measurement regions, compares this average intensity with a reference value, compares deviations between the measurement regions, and, based on this comparison, when necessary generates an error signal. In addition, a cooling curve is calculated and used as a reference to compensate for the difference in the amount of radiation of glass products due to different cooling times.

However, the system disclosed in the latter WO 2004/011935 may generate error signals even when there is a change in the amount of radiation which is not brought about due to a change in the forming process, but is due instead to changes in the various conditions and parameters, such as, among others, the ambient temperature, ambient humidity, production speed, cooling air temperature, cooling air humidity, glass material composition, camera settings, smoke and dirt in the air, pollution of the optics, and container weight.

These conditions and parameters can drastically alter the measured radiation intensities depending on, for example, whether operating at day or night, different seasons, the production location, and/or the forming machine.

Consequently, an operator should always be present to monitor the measurement results and the generated error signals carefully, to check the conditions and parameters, and to adjust reference values in order to compensate for continuously changing conditions and parameters. From a practical point of view this is a very undesirable requirement, since labor costs are high and the forming process occurs in an extremely hot and noisy environment where labor conditions are quite unfavorable.

Another disadvantage of the system disclosed in WO 2004/011935 is that when starting the production of a glass container which has been produced earlier, the above mentioned conditions and parameters may have been changed, in which case the reference values and/or cooling curves used for the previous production may not be useful for the current production. In such a case, each time, a new reference and/or a cooling curve is required, which will lengthen the start up time and is therefore not desirable.

A primary object of the present invention is to provide a method for monitoring a glass container forming process which is independent of the above mentioned conditions and parameters and with which it is possible to produce glass containers with a high and constant quality.

Another object of the present invention is to generate a unique master reference which serves as a quality reference for the type of container produced, in order to produce glass containers at each forming station with the same quality depicted by this master reference and to decrease the time necessary to start up the forming process. This unique master reference may be stored and used to monitor and control the same forming machine or another forming machine producing the same particular glass container type at a different location.

Yet a further object of the present invention is to eliminate the requirement of having an operator monitoring the process constantly by instead controlling the forming machine automatically.

The present invention relates to a method for monitoring a glass container forming process generally as disclosed in WO 2004/011935 and as defined in the preamble of claim 1. The method comprises the steps of measuring radiation emitted by hot glass containers immediately after the forming machine with a measurement unit, for example a camera, sensitive to the radiation emitted by the hot glass containers. The glass container image generated by the camera is arranged in a finite number of image lines, each image line having a finite number of pixels.

The method for monitoring a glass container forming process according to the present invention is defined in the characterising clause of claim 1.

The above mentioned problem of changing conditions and parameters is compensated for by dividing the line radiation intensities with the total radiation intensity. This can be explained as follows. When, for example, the radiation of a hot glass container is partly absorbed by some smoke in the air, the line radiation intensities gets lower and the total intensity gets lower. However, the result of dividing the line radiation intensities by the total intensity will not change (the intensity-ratio curve will not change). Obviously, this would not have been the case when, as in the prior art, absolute values of the intensity are used.

Glass containers originating from forming stations close to the measurement unit when compared to containers from forming stations further away travel a shorter distance to the measurement unit taking less time, and thus cool down less and consequently will have a higher temperature. In the prior art, a cooling curve is used to compensate for these different cooling times. However, this cooling curve is based on absolute intensity values, and is therefore sensitive to changes in above-mentioned conditions and parameters. According to the present invention, the intensity-ratio curve of the hot glass containers compensates for the temperature of the glass container. When a glass container from a forming station further away (lower temperature) has the same glass distribution as a glass container from a forming station closer to the measurement unit, the intensity-ratio curves from both glass containers will be the same.

The intensity-ratio curve of a hot glass container can further be compared with a master reference. The master reference is unique for each particular glass container type, and thus serves as a quality measure for each particular glass container type. In order to acquire this unique master reference, a preferred embodiment of method according to the present invention further comprises the following step:

> **d.** determining a master reference curve by averaging the intensity-ratio curves over a predetermined number of hot glass containers from all or a selected number of forming stations.

Averaging the intensity-ratio curves over a number of hot glass containers includes summing the intensity-ratio curves of a number of glass containers and dividing this sum by the number of glass containers. Glass containers may be averaged over a period of time, from one or more selected stations, over a number of production cycles, or over just one production cycle.

One may obtain various master reference curves with different forming machine settings, from which the one having the best performance, producing the best quality of glass containers, may be selected. This master reference curve can be saved in order to be used when producing the same container type at a later time, either on the same forming machine or on a different forming machine. It can also be used to analyze and compare the current production performance with a past production. If desired, the master reference curve can be continuously updated which may result in an even better quality being achieved for the same container.

Another preferred embodiment of method according to the present invention comprises the following step:

> e. determining a relative difference curve for each hot glass container by subtracting said master reference curve from said intensity-ratio curve of each hot glass container and dividing the result by said master reference curve.

The relative difference curve easily shows how much and where the intensity-ratio curve of a glass container deviates from the master reference curve. The relative difference curve can be displayed for each forming station in order to show the quality of the glass containers produced at the forming station. When the quality of a produced glass container is high, the relative difference curve will be close to zero. When the relative difference curves of all containers from all stations are close to zero, the quality of all containers produced by the forming machine will be high and substantially equal.

Another preferred embodiment of method according to the present invention comprises the following steps:

> f. comparing said relative difference curve with a predetermined tolerance curve.

g. generating an alarm signal if said relative difference curve exceeds said tolerance curve in at least one point.

By comparing the relative difference curve with a tolerance curve and generating an alarm signal if the difference exceeds the tolerance curve, one can easily determine whether the quality of glass containers produced by a forming station is acceptable or if it has degraded to such a degree that the container is of an inferior quality and therefore unacceptable.

The tolerance curve may be constant, tolerating the same amount of deviation for every location on said container. However, the values of the tolerance curve may also be dependant on the location on said container. By doing so, one is for instance able to allow less deviation from the master reference curve for one or more areas of the container where the glass thickness is critical. Furthermore, the tolerance values may be positive as well as negative.

A control unit controls the forming process of each forming station by a number of process parameters. In order to control the forming process automatically, another preferred embodiment of method according to the present invention comprises the following step:

h. sending said relative difference curve of each hot glass container to said control unit.

By sending the relative difference curves of glass containers from each forming station to the control unit, the adjustment of the process may be performed automatically and shortly after the detection of a change or an error in the forming process. The adjustment will occur in such a manner that the relative difference curves substantially decreases to zero.

The measurement may be carried out at any wavelength at which a hot glass container emits radiation. Nevertheless, since the amount of radiation of container glass not only depends on the glass temperature but also on the glass thickness for wavelengths smaller than 3.0 microns, the measurement will be more accurate at wavelengths smaller than 3.0 microns, especially when analyzing relatively thicker glass containers. Therefore, a preferred embodiment method according to the present invention is characterized in that said measurement occurs for wavelengths of between 0.7 and 3.0 microns.

The present invention also relates to an analytical system for monitoring a glass container forming process generally as disclosed in WO 2004/011935 and as defined in the preamble of claim 7. The system comprises at least one measurement unit to measure radiation emitted by each hot glass container immediately after the forming machine. The measurement unit may comprise a line-scan or area camera sensitive to the radiation emitted by the hot glass containers. The glass container image generated by the camera is arranged in a finite number of image lines, with each image line having a finite number of pixels. A processor unit provides calculations, comparisons, and communications with other units.

The processor unit of the analytical system according to the present invention is defined in the characterizing clause of claim 7.

The processor unit is programmed such that it performs the above mentioned operation in order to make the measured values of intensities independent not only of changes in parameters and conditions of the environment, process, and measuring equipment, but also independent of the forming station where a hot glass container originates from.

A further preferred embodiment of the analytical system according to the present invention is characterized in that the processor unit is further programmed to carry out the following step:

d. determining a master reference curve by averaging the intensity-ratio curves over a predetermined number of hot glass containers from all or a selected number of forming stations.

By summing the intensity-ratio curves of a number of hot glass containers and dividing the sum by the number of containers, an average intensity-ratio curve is acquired which is unique for a particular type of glass container. The average intensity-ratio curve may serve as a master reference for the quality of glass containers of that particular type. It can also be utilized for another forming machine at a different location when producing the same type of glass container with the same quality requirements.

Another preferred embodiment of analytical system according to the present invention is **characterized in that** the processor unit is further programmed to carry out the following step:

e. determining a relative difference curve for each hot glass container by subtracting said master reference curve from said intensity-ratio curve of each hot glass container and dividing the result by said master reference curve.

By determining the relative difference curve, the quality of a hot glass container may be analyzed and the possible cause of a deficiency in the forming process may be indicated. Doing this, one can easily see how much and where the intensity ratio curve of a hot glass container deviates from the master reference curve. The relative difference curve can be displayed for each forming station in order to show the quality of the produced glass containers and to show the performance of the forming process. When the quality of a produced glass container is high, the relative difference curve will be zero or negligibly small.

Another preferred embodiment of analytical system according to the present invention is **characterized in that** the processor unit is further programmed to carry out the following step:

    f. comparing said relative difference curve of each hot glass container with a predetermined tolerance curve;

    g. generating an alarm signal if said relative difference curve exceeds said tolerance curve in at least one point.

Doing this, it can easily be determined whether the quality of the produced glass containers at a forming station is acceptable or not. The alarm signal may be used, for instance, to reject glass containers which have an unacceptable quality. The tolerance curve may be constant or it may be variable dependant on the location on the glass container.

Still another preferred embodiment of analytical system according to the present invention is **characterized in that** the processor unit is further programmed to carry out the following step:

    h. sending said relative difference curve of each hot glass container to the forming control unit.

The processor unit sends the relative difference curve of each glass container to the forming control unit, and the forming control unit, when necessary, adjusts one or more process parameters. This way, an automatic adjustment of process parameters is feasible shortly after the detection of an error or any detectable deficiency.

The measurement unit may be sensitive to any wavelength at which a hot glass container emits radiation. Nevertheless, since the amount of radiation of container glass depends on the glass temperature and the glass thickness for wavelengths smaller than 3.0 microns, the measurement will be more accurate at wavelengths smaller than 3.0 microns, especially when analyzing relatively thicker glass containers. Therefore, a preferred embodiment of analytical system according to the present invention is **characterized in that** the measurement unit is sensitive to wavelengths of between 0.7 and 3.0 microns. More specifically, the measurement unit comprises a Short Wave Infra Red (SWIR) camera, for example a 512 or 1024 pixels line-scan or area SWIR camera.

The invention will be explained in more detail with reference to the appended drawings in which:

    FIG. 1 shows a schematic view of a forming machine and an embodiment of the analytical system,
    FIG. 2a shows an image of a glass container,
    FIG. 2b shows the line radiation intensities for the glass container shown in FIG. 1,
    FIG. 2c shows an intensity-ratio curve for the glass container shown in FIG. 1,
    FIG. 2d shows a master reference curve for the glass container shown in FIG. 1,
    FIG. 2e shows a master reference curve together with the intensity-ratio curve for the glass container shown in FIG. 1, and
    FIG. 2f shows a relative difference curve for the glass container shown in FIG. 1.

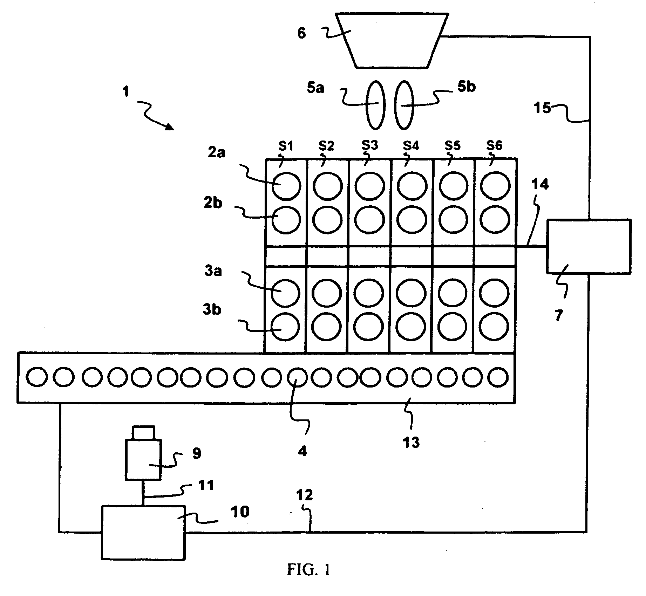

FIG 1. shows an embodiment of the system where the glass container forming machine (1) contains six independent sections (S1), (S2), ... (S6), each of which contains two forming stations (2a) and (2b). In one production cycle, the forming machine (1) produces twelve glass containers (4). Two molten glass gobs (5a) and (5b) are formed at the same moment by the feeder unit (6) and are loaded into the so-called blank moulds (2a) and (2b). Each section (S1), (S2), ... (S6) of the forming machine (1) in this embodiment contains two blank moulds (2a) and (2b) in which pre-containers or parisons are formed by pressing or blowing depending on the process type (press-blow or blow-blow). The formed parisons are transferred to the so-called blow moulds (3a) and (3b) where the parisons are blown into the final shape of the glass containers (4). The mechanisms of the forming machine (1) and the feeder unit (6) are controlled by the control unit (7) through lines (14) and (15), respectively. The glass containers (4) are transported by a conveyor belt (13) through a measurement unit (9) which takes images of the hot glass containers (4) and sends these images to a processor unit (10) through a line (11). Although in this embodiment one measurement unit (9) is used, the number of measurement units (9) may be increased depending on the circumstances and the accuracy to be achieved. However, even with one measurement unit, the achieved accuracy is fairly high.

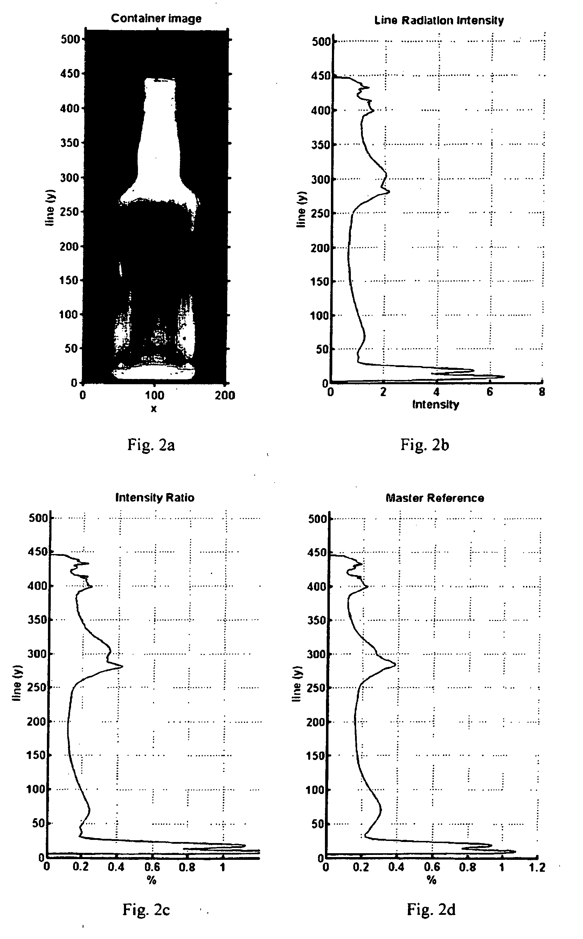

The measurement unit (9), an area camera in this embodiment, is preferably sensitive to Short Wave Infra Red (SWIR) radiation. The image area of the camera within which the image of the hot glass container (4) shown in FIG. 2a is situated contains a finite number of image-lines, preferably 512 image-lines, with each image-line containing a finite number of pixels, preferably 200 pixels.

The processor unit (10) determines for each glass container (4) the total intensity by summing the intensities of all the pixels in the container image. The total intensity of the container shown in Fig. 2a has a value of 553. Next, the processor unit (10) determines the line radiation intensities by summing for each image-line the intensities of all 200 pixels. The line radiation intensities belonging to the glass container image of FIG. 2a is shown in FIG. 2b. Now, the processor unit (10), determines the intensity-ratio curve by dividing the line radiation intensities by the total intensity, as shown hereunder:

$$I_{tot, s} = \Sigma I_{x,y, s} \ (x= 1, 2, ... 200, y= 1, 2, ... 512)$$

$$I_{y, s} = \Sigma I_{x,y, s} \ (x= 1, 2, ... 200)$$

$$I_{ratio,y, s} = (I_{y, s} / I_{tot, s}) * 100\%$$

Where:

$I_{tot, s}$ = the total intensity value of a container image, originating from station (s),

$I_{x,y, s}$ = the intensity value of pixel (x,y) of the container image, originating from station (s) with (y) representing an image-line containing 200 (x) pixels, x=1..200, y=1..512, s=1..12,

$I_{y, s}$ = the radiation intensity value for image-line (y) of a container image, originating from station (s),

$I_{ratio, y, s}$ = the intensity-ratio value for image-line (y) of a container image, originating from station (s).

The intensity-ratio values are expressed in percentages for clarity. The intensity-ratio curve depicted in FIG. 2c belongs to the glass container shown in FIG. 2a. Obviously, the order in which these steps occur can be changed as long as the same results are achieved. One can easily see that, for example, an attenuation $\alpha$ of the radiation received from the glass container (4) caused by an ambient parameter (for example smoke in the air) has no influence on the intensity-ratio curve:

$$I_{ratio,y, s} = (\alpha I_{y, s} / \alpha I_{tot, s}) * 100\% = (I_{y, s} / I_{tot, s}) * 100\%$$

Next, the processor unit (10) determines a master reference curve by averaging intensity-ratio curves from a number of glass containers (4) from all or certain selected forming stations. This master reference curve is unique for the glass container type produced.

The values of the master reference curve are derived as illustrated below:

$$I_{reference, y} = (\Sigma I_{ratio, y, s}) / N$$

Where:

$I_{reference,y}$ = the master reference curve value for line (y).

N = number of glass containers (4) taken into account

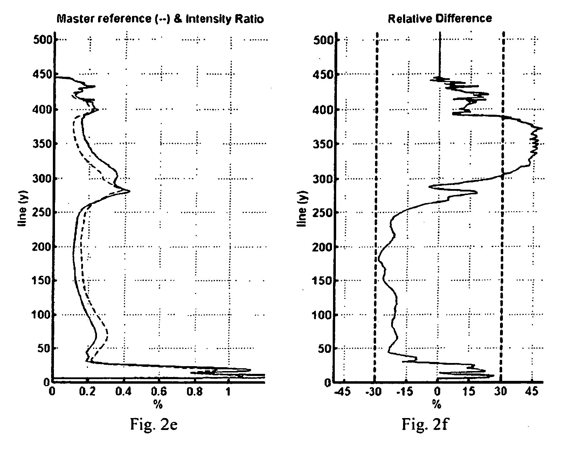

The master reference curve may be stored and used later to decrease the time necessary to start up the production of the particular container (4) on the same or on another forming machine. The master reference curve belonging to the container type in this example is shown in FIG. 2d. In FIG. 2e, the master reference curve is shown together with the intensity-ratio curve of FIG. 2c.

The processor unit (10) next determines the relative difference curve by subtracting the master reference curve from the intensity-ratio curve and dividing the difference by the master reference curve. This is illustrated hereunder:

$$\Delta I_{s, y} = ( (I_{ratio, y, s} - I_{reference, y}) / I_{reference, y}) * 100\%$$

Where:

$\Delta I_{s,y}$ = the relative difference value at line (y) of a container image originating from the station (s).

The relative difference curve shows how much and where the intensity-ratio curve of a glass container deviates from the master reference curve. The processor unit (10) may display on a connected monitor (not shown) for each forming station the relative difference curve in order to show the quality of the glass containers produced at the forming station. In FIG. 2f, the relative difference curve is shown for the glass container of FIG. 2a with the corresponding intensity-ratio curve shown in FIG. 2c.

In this specific example the relative difference curve in FIG. 2f shows a positive deviation in the upper part of the container and a negative deviation in the lower part of the container, indicating too much glass in the upper part of the container and too little glass in the lower part of the container. The relative difference curve will be close to zero at every point for high quality glass containers.

Subsequently, the processor unit (10) compares the relative difference curve with predetermined tolerance curves and generates an alarm signal if a relative difference value exceeds the corresponding tolerance value. This is illustrated hereunder: Alarm if:

$$\Delta I_{s,y} < I_{T-,y} \text{ or } \Delta I_{s,y} > I_{T+,y}$$

Where:

$I_{T-,y}$ = the negative tolerance value for line (y);
$I_{T+,y}$ = the positive tolerance value for line (y).

The alarm signal may, for example, be used in order to reject glass containers which have an unacceptable quality. In FIG. 2f the negative tolerance values are set at -30% and the positive tolerance values are set at +30%. In FIG. 2f an alarm signal is generated because the relative difference values for line 300 through line 380 exceed the positive tolerance values.

In order to adjust the forming process automatically, the processor unit (10) may send the relative difference curve from each forming station to the control unit (7) over line (12). The control unit (7) adjusts the appropriate process parameters until the relative difference curve for each forming station is close to zero. This is then achieved without the need to have an operator monitoring the process continuously.

The processor unit (10) is synchronized with the forming machine (1) and with conveyor belt (13) in such a way that processor unit (10) knows from which forming station each glass container (4) originates.

The embodiment described above is intended solely to serve as an example, and in no way is intended to restrict the invention, as defined in the appended claims. A person skilled in the art given knowledge of this invention will rapidly be able to accomplish other embodiments.

**Claims**

1. A method for monitoring a glass container forming process wherein said forming process is accomplished by a forming machine (1) which contains multiple independent sections (S1, S2,..., S6) each having at least one forming station (2a, 2b, 3a, 3b) at which glass containers (4) are formed, comprising the steps of measuring radiation emitted by successive hot glass containers (4) from the forming stations (2a, 2b, 3a, 3b) immediately after said forming machine (1) with a measurement unit (9) sensitive to the radiation emitted by the hot glass containers (4), wherein the measurement unit (9) generates an image of each hot glass container (4) which is arranged within an associated image area defined by a finite number of image lines, each image line having a finite number of pixels, **characterized in that** said method further comprises the following steps for each glass container (4) measured from each forming station (2a, 2b, 3a, 3b):

   a. determining a total radiation intensity for each glass container (4) by summing the intensity values of all the pixels in all the image lines for such glass container (4);
   b. determining a line radiation intensity for each image line for each glass container (4) by summing the intensity values of all the pixels in such image line for such glass container (4); and
   c. determining an intensity-ratio curve for each glass container (4) by dividing the line radiation intensities for each image line of such glass container (4) by the total radiation intensity for such glass container (4), thereby to compensate for changes in the amount of radiation emitted by the glass containers (4) resulting from changes in conditions and parameters to which the glass containers (4) are subjected as they travel from their forming stations to the measurement unit (9).

2. A method according to Claim 1, **characterised in that** said method further comprises the following step in order to generate a reference, unique to the type of container to be formed, with which glass containers (4) from each forming station (2a, 2b, 3a, 3b) are compared:

d. determining a master reference curve by averaging corresponding points of intensity-ratio curved over a predetermined number of said glass containers (4) originating from all or from a selected number of forming stations (2a, 2b, 3a, 3b).

3. A method according to Claim 2, **characterised in that** said method further comprises the following step in order to generate information about the difference between the glass containers (4) produced in a forming station (2a, 2b, 3a, 3b) with said master reference curve:

e. determining a relative difference curve for each glass container (4) from each forming station (2a, 2b, 3a, 3b) by subtracting said master reference curve from said intensity-ration curve and dividing the result by said master reference curve.

4. A method according to Claim 3, **characterised in that** said method further comprises the following step:

f. comparing said relative difference curve of each hot glass container (4) with predetermined tolerance curves;
g. generating an alarm signal if said relative difference curve exceeds said tolerance curves in at least one point.

5. A method according to Claim 3 or 4, **characterised in that** said method further comprises the following step in order to control the forming process automatically:

h. sending said relative difference curve of each hot glass container (4) to a forming process-control unit (7).

6. A method according to any of the preceding claims, **characterised in that** said measurement occurs for wavelengths of between 0.7 and 3.0 microns.

7. A system for monitoring a glass container forming process, wherein said forming process is accomplished by a forming machine (1) which is controlled by a control unit (7) and which contains multiple independent sections (S1, S2,..., S6) each having at least one forming station (2a, 2b, 3a, 3b) at which glass containers (4) are formed, comprising at least one measurement unit (9) sensitive to the radiation emitted by successive hot glass containers (4) from the forming stations (2a, 2b, 3a, 3b) immediately after said forming machine (1), generating images of each hot glass container (4) arranged within an associated image area defined by a finite number of image lines, each image line having a finite number of pixels, and a processor unit (10) to provide calculations, comparisons and communications with other units, **characterized in that** said processor unit (10) is further programmed to carry out the following steps for each glass container (4) measured from each forming station (2a, 2b, 3a, 3b):

a. determining a total radiation intensity for each glass container (4) by summing the intensity values of all the pixels in all the image lines for such glass container (4);
b. determining a line radiation intensity for each image line for each glass container (4) by summing the intensity values of all the pixels in such image line for such glass container (4); and
c. determining an intensity-ratio curve for each glass container (4) by dividing the line radiation intensities for each image line of such glass container (4) by the total radiation intensity for such glass container (4), thereby to compensate for changes in the amount of radiation emitted by the glass containers (4) resulting from changes in conditions and parameters to which the glass containers (4) are subjected as they travel from their forming stations (2a, 2b, 3a, 3b) to the measurement unit (9).

8. A system according to Claim 7, **characterised in that**, said processor unit (10) is further programmed to carry out the following step:

d. determining a master reference curve by averaging corresponding points of intensity-ratio curves over a predetermined number of said glass containers (4) originating from all or from a selected number of forming stations (2a, 2b, 3a, 3b).

9. A system according to Claim 8, **characterised in that**, said processor unit (10) is further programmed to carry out the following steps:

e. determining a relative difference curve for each hot glass container (4) by subtracting said master reference curve from said intensity-ratio curve of each hot glass container (4) and dividing the result by said master reference curve.

**10.** A system according to Claim 9, **characterised in that**, said processor unit (10) is further programmed to carry out the following steps:

> f. comparing said relative difference curve of each hot glass container (4) with predetermined tolerance curves.
> g. generating an alarm signal if said relative difference curve exceeds said tolerance curves in at least one point.

**11.** A system according to Claim 9 or 10, **characterised in that**, said processor unit (10) is further programmed to carry out the following step in order to control the form ing process automatically:

> h. sending said relative difference curve of each hot glass container (4) to said control unit (7).

**12.** A system according to any of the Claims 7-11, **characterised in that** said measurement unit (9) is sensitive to wavelengths of between 0.7 and 3.0 microns.

**13.** A system according to Claim 12, **characterised in that**, said measurement unit (9) comprises a Short Wave Infra Red (SWIR) camera.

**Patentansprüche**

**1.** Verfahren zum Überwachen eines Glasbehälterformprozesses, wobei der Formprozess durch eine Formmaschine (1) erfolgt, die mehrere unabhängige Abschnitte (51, 52..., 56) hat, von denen jeder mindestens eine Formstation (2a, 2b, 3a, 3b) aufweist, an der Glasbehälter (4) geformt werden, umfassend die Schritte:

Ermitteln der von nacheinander aus den Formstationen (2a, 2b, 3a, 3b) kommenden Glasbehältern (4) abgegebenen Strahlung unmittelbar nach der Formmaschine (1) mit einer Messeinheit (9), die auf die von den heißen Glasbehältern (4) abgegebene Strahlung reagiert, wobei die Messeinheit (9) ein Bild jedes heißen Glasbehälters (4) erzeugt, das innerhalb eines zugeordneten Bildbereiches angeordnet wird, der von einer endlichen Anzahl von Bildlinien definiert wird, wobei jede Bildlinie eine endliche Anzahl von Pixeln hat, **dadurch gekennzeichnet, dass** das Verfahren weiterhin für jeden von jeder Formstation (2a, 2b, 3a, 3b) kommenden gemessenen Glasbehälter (4) die folgenden Schritte umfasst:

> a. Bestimmen einer Gesamtstrahlungsintensität für jeden Glasbehälter (4) durch Aufsummieren der Intensitätswerte aller Pixel in allen Bildzeilen für einen solchen Glasbehälter (4);
> b. Bestimmen einer Linienstrahlungsintensität für jede Bildlinie für jeden Glasbehälter (4) durch Aufsummieren der Intensitätswerte aller Pixel in jeder solchen Bildlinie für jeden solchen Glasbehälter (4); und
> c. Bestimmen einer Intensitätsverhältniskurve für jeden Glasbehälter (4) durch Division der Linienstrahlungsintensitäten für jede Bildlinie jedes solchen Glasbehälters (4) durch die Gesamtstrahlungsintensität für jeden solchen Glasbehälter (4), um Änderungen in der Strahlungsmenge, die von den Glasbehältern (4) abgegeben wird, auszugleichen, die von Änderungen in den Bedingungen und Parametern herrühren, denen die Glasbehälter (4) unterworfen sind, wenn sie von ihren Formstationen zur Messeinheit (19) laufen.

**2.** Verfahren nach Anspruch 1, **dadurch gekennzeichnet, dass** das Verfahren weiterhin den folgenden Schritt umfasst, um eine für den zu formenden Behältertyp einzigartige Referenz zu erzeugen, mit der von jeder Formstation (2a, 2b, 3a, 3b) kommende Glasbehälter (4) verglichen werden:

> d. Bestimmen einer Original-Referenzkurve durch Mitteln entsprechender Punkte von Intensitätsverhältniskurven über eine vorbestimmte Anzahl der Glasbehälter (4), die von allen oder von einer ausgewählten Anzahl von Formstationen (2a, 2b, 3a, 3b) kommen.

**3.** Verfahren nach Anspruch 2, **dadurch gekennzeichnet, dass** das Verfahren weiterhin den folgenden Schritt umfasst, um Informationen über den Unterschied zwischen den Glasbehältern (4) zu erstellen, die in einer Formstation (2a, 2b, 3a, 3b) mit der Original-Referenzkurve hergestellt wurden:

> e. Bestimmen einer Relativdifferenzkurve für jeden Glasbehälter (4) aus jeder Formstation (2a, 2b, 3a, 3b) durch Subtrahieren der Original-Referenzkurve von der Intensitätsverhältniskurve und Dividieren des Ergebnisses durch die Original-Referenzkurve.

**4.** Verfahren nach Anspruch 3, **dadurch gekennzeichnet, dass** das Verfahren weiterhin die folgenden Schritte umfasst:

> f. Vergleichen der Relativdifferenzkurve für jeden heißen Glasbehälter (4) mit vorbestimmten Toleranzkurven;
> g. Erzeugen eines Alarmsignals, falls die Relativdifferenzkurve die Toleranzkurven in mindestens einem Punkt übersteigt.

**5.** Verfahren nach Anspruch 3 oder 4, **dadurch gekennzeichnet, dass** das Verfahren weiterhin den folgenden Schritt umfasst, um den Formprozess automatisch zu steuern:

> h. Senden der Relativdifferenzkurve für jeden heißen Glasbehälter (4) an eine Formprozesssteuereinheit (7).

**6.** Verfahren nach einem der vorangegangenen Ansprüche, **dadurch gekennzeichnet, dass** die Messung für Wellenlängen zwischen 0,7 und 3,0 Mikrometern erfolgt.

**7.** System zum Überwachen eines Glasbehälterformprozesses, wobei der Formprozess durch eine Formmaschine (1) erfolgt, die von einer Steuereinheit (7) gesteuert wird und mehrere unabhängige Abschnitte (51, 52..., 56) hat, von denen jeder mindestens eine Formstation (2a, 2b, 3a, 3b) aufweist, an der Glasbehälter (4) geformt werden, umfassend mindestens eine Messeinheit (9), die auf die von nacheinander aus den Formstationen (2a, 2b, 3a, 3b) kommenden Glasbehältern (4) abgegebene Strahlung unmittelbar nach der Formmaschine (1) reagiert, zum Erzeugen von Bildern für jeden heißen Glasbehälter (4), die innerhalb eines zugeordneten Bildbereiches angeordnet sind, der von einer endlichen Anzahl von Bildlinien definiert wird, wobei jede Bildlinie eine endliche Anzahl von Pixeln hat, und mit einer Prozessoreinheit (10) zum Ausführen von Berechnungen, Vergleichen und Kommunizieren mit anderen Einheiten, **dadurch gekennzeichnet, dass** die Prozessoreinheit (10) weiterhin programmiert ist, um die folgenden Schritte für jeden von jeder Formstation (2a, 2b, 3a, 3b) gemessenen Glasbehälter (4) auszuführen:

> a. Bestimmen einer Gesamtstrahlungsintensität für jeden Glasbehälter (4) durch Aufsummieren der Intensitätswerte aller Pixel in allen Bildzeilen für einen solchen Glasbehälter (4);
> b. Bestimmen einer Linienstrahlungsintensität für jede Bildlinie für jeden Glasbehälter (4) durch Aufsummieren der Intensitätswerte aller Pixel in jeder solchen Bildlinie für jeden solchen Glasbehälter (4); und
> c. Bestimmen einer Intensitätsverhältniskurve für jeden Glasbehälter (4) durch Division der Linienstrahlungsintensitäten für jede Bildlinie jedes solchen Glasbehälters (4) durch die Gesamtstrahlungsintensität für jeden solchen Glasbehälter (4), um Änderungen in der Strahlungsmenge, die von den Glasbehältern (4) abgegeben wird, auszugleichen, die von Änderungen in den Bedingungen und Parametern herrühren, denen die Glasbehälter (4) unterworfen sind, wenn sie von ihren Formstationen zur Messeinheit (19) laufen.

**8.** System nach Anspruch 7, **dadurch gekennzeichnet, dass** die Prozesseinheit (10) weiterhin programmiert ist, um den folgenden Schritt auszuführen:

> d. Bestimmen einer Original-Referenzkurve durch Mitteln entsprechender Punkte von Intensitätsverhältniskurven über eine vorbestimmte Anzahl der Glasbehälter (4), die von allen oder von einer ausgewählten Anzahl von Formstationen (2a, 2b, 3a, 3b) kommen.

**9.** System nach Anspruch 8, **dadurch gekennzeichnet, dass** die Prozesseinheit (10) weiterhin programmiert ist, um die folgenden Schritte auszuführen:

> e. Bestimmen einer Relativdifferenzkurve für jeden heißen Glasbehälter (4) durch Subtrahieren der Original-Referenzkurve von der Intensitätsverhältniskurve und durch Dividieren des Ergebnisses durch die Original-Referenzkurve.

**10.** System nach Anspruch 9, **dadurch gekennzeichnet, dass** die Prozessoreinheit (10) weiterhin programmiert ist, um die folgenden Schritte auszuführen:

> f. Vergleichen der Relativdifferenzkurve für jeden heißen Glasbehälter (4) mit vorbestimmten Toleranzkurven;
> g. Erzeugen eines Alarmsignals, falls die Relativdifferenzkurve die Toleranzkurven in mindestens einem Punkt übersteigt.

**11.** System nach Anspruch 9 oder 10, **dadurch gekennzeichnet, dass** die Prozessoreinheit weiterhin programmiert ist, um den folgenden Schritt auszuführen, um den Formprozess automatisch zu steuern.

h. Senden der Relativdifferenzkurve für jeden heißen Glasbehälter (4) an die Steuereinheit (7).

**12.** System nach einem der Ansprüche 7 bis 11, **dadurch gekennzeichnet, dass** die Messeinheit (9) auf Wellenlängen zwischen 0,7 und 3,0 Mikrometern reagiert.

**13.** System nach Anspruch 12, **dadurch gekennzeichnet, dass** die Messeinheit (9) eine Kurzwelleninfrarot (SWIR)-Kamera umfasst.

**Revendications**

**1.** Procédé de surveillance d'un processus de formage de récipient en verre dans lequel ledit processus de formage est accompli par une machine de formage (1) qui contient de multiples sections indépendantes (S1, S2, ..., S6) comportant chacune au moins un poste de formage (2a, 2b, 3a, 3b) au niveau duquel les récipients en verre (4) sont formés, comprenant les étapes de mesure d'un rayonnement émis par des récipients en verre (4) chauds successifs provenant des postes de formage (2a, 2b, 3a, 3b) immédiatement après ladite machine de formage (1) par une unité de mesure (9) sensible au rayonnement émis par les récipients en verre (4) chauds, dans lequel l'unité de mesure (9) génère une image de chaque récipient en verre (4) chaud qui est agencé dans une zone d'image associée définie par un nombre fini de lignes d'image, chaque ligne d'image comportant un nombre fini de pixels, **caractérisé en ce que** ledit procédé comprend en outre les étapes suivantes pour chaque récipient en verre (4) mesuré provenant de chaque poste de formage (2a, 2b, 3a, 3b) :

a. de détermination d'une intensité de rayonnement totale pour chaque récipient en verre (4) en sommant les valeurs d'intensité de tous les pixels dans toutes les lignes d'image pour ce récipient en verre (4) ;
b. de détermination d'une intensité de rayonnement de ligne pour chaque ligne d'image pour chaque récipient en verre (4) en sommant les valeurs d'intensité de tous les pixels dans cette ligne d'image pour ce récipient en verre (4) ; et
c. de détermination d'une courbe de rapport d'intensités pour chaque récipient en verre (4) en divisant les intensités de rayonnement de ligne pour chaque ligne d'image de ce récipient en verre (4) par l'intensité de rayonnement totale pour ce récipient en verre (4), pour compenser de ce fait des variations de la quantité du rayonnement émis par les récipients en verre (4) résultant de changements des conditions et des paramètres auxquels les récipients en verre (4) sont soumis alors qu'ils se déplacent de leurs postes de formage vers l'unité de mesure (9).

**2.** Procédé selon la revendication 1, **caractérisé en ce que** ledit procédé comprend en outre l'étape suivante afin de générer une référence, propre au type de récipient à former, à laquelle les récipients en verre (4) provenant de chaque poste de formage (2a, 2b, 3a, 3b) sont comparés :

d. de détermination d'une courbe de référence maître en moyennant des points correspondants de courbes de rapport d'intensités sur un nombre prédéterminé desdits récipients en verre (4) provenant de la totalité ou d'un nombre sélectionné de postes de formage (2a, 2b, 3a, 3b).

**3.** Procédé selon la revendication 2, **caractérisé en ce que** ledit procédé comprend en outre l'étape suivante afin de générer des informations concernant la différence entre les récipients en verre (4) produits dans un poste de formage (2a, 2b, 3a, 3b) avec ladite courbe de référence maître :

e. de détermination d'une courbe de différences relatives pour chaque récipient en verre (4) provenant de chaque poste de formage (2a, 2b, 3a, 3b) en soustrayant ladite courbe de référence maître de ladite courbe de rapport d'intensités et en divisant le résultat par ladite courbe de référence maître.

**4.** Procédé selon la revendication 3, **caractérisé en ce que** ledit procédé comprend en outre l'étape suivante :

f. de comparaison de ladite courbe de différences relatives de chaque récipient en verre (4) chaud avec des courbes de tolérance prédéterminées ;
g. de génération d'un signal d'alarme si ladite courbe de différences relatives dépasse lesdites courbes de tolérance en au moins un point.

5. Procédé selon la revendication 3 ou 4, **caractérisé en ce que** ledit procédé comprend en outre l'étape suivante afin de commander le processus de formage automatiquement :

> h. d'envoi de ladite courbe de différences relatives de chaque récipient en verre (4) chaud à une unité de commande de processus de formage (7).

6. Procédé selon l'une quelconque des revendications précédentes, **caractérisé en ce que** ladite mesure est effectuée pour des longueurs d'onde entre 0,7 et 3,0 microns.

7. Système pour surveiller un processus de formage de récipient en verre, dans lequel ledit processus de formage est effectué par une machine de formage (1) qui est commandée par une unité de commande (7) et qui contient de multiples sections indépendantes (S1, S2, ..., S6), comportant chacune au moins un poste de formage (2a, 2b, 3a, 3b) au niveau duquel les récipients en verre (4) sont formés, comprenant au moins une unité de mesure (9) sensible au rayonnement émis par des récipients en verre (4) chauds successifs provenant des postes de formage (2a, 2b, 3a, 3b) immédiatement après ladite machine de formage (1), générant des images de chaque récipient en verre (4) chaud agencé dans une zone d'image associée définie par un nombre fini de lignes d'image, chaque ligne d'image comportant un nombre fini de pixels, et une unité de processeur (10) pour effectuer des calculs, des comparaisons et des communications avec d'autres unités, **caractérisé en ce que** ladite unité de processeur (10) est en outre programmée pour effectuer les étapes suivantes pour chaque récipient en verre (4) mesuré provenant de chaque poste de formage (2a, 2b, 3a, 3b) :

> a. de détermination d'une intensité de rayonnement totale pour chaque récipient en verre (4) en sommant les valeurs d'intensité de tous les pixels dans toutes les lignes d'image pour ce récipient en verre (4),
> b. de détermination d'une intensité de rayonnement de ligne pour chaque ligne d'image pour chaque récipient en verre (4) en sommant les valeurs d'intensité de tous les pixels dans cette ligne d'image pour ce récipient en verre (4), et
> c. de détermination d'une courbe de rapport d'intensités pour chaque récipient en verre (4) en divisant les intensités de rayonnement de ligne pour chaque ligne d'image de ce récipient en verre (4) par l'intensité de rayonnement totale pour ce récipient en verre (4), pour compenser de ce fait des variations de la quantité du rayonnement émis par les récipients en verre (4) résultant de changements des conditions et des paramètres auxquels les récipients en verre (4) sont soumis alors qu'ils se déplacent de leur poste de formage (2a, 2b, 3a, 3b) vers l'unité de mesure (9).

8. Système selon la revendication 7, **caractérisé en ce que** ladite unité de processeur (10) est en outre programmée pour effectuer l'étape suivante :

> d. de détermination d'une courbe de référence maître en moyennant des points correspondants de courbes de rapport d'intensités sur un nombre prédéterminé desdits récipients en verre (4) provenant de la totalité ou d'un nombre sélectionné de postes de formage (2a, 2b, 3a, 3b).

9. Système selon la revendication 8, **caractérisé en ce que** ladite unité de processeur (10) est en outre programmée pour exécuter l'étape suivante :

> e. de détermination d'une courbe de différences relatives pour chaque récipient en verre (4) chaud en soustrayant ladite courbe de référence maître de ladite courbe de rapport d'intensités de chaque récipient en verre (4) chaud et en divisant le résultat par ladite courbe de référence maître.

10. Système selon la revendication 9, **caractérisé en ce que** ladite unité de processeur (10) est en outre programmée pour exécuter les étapes suivantes :

> f. de comparaison de ladite courbe de différences relatives de chaque récipient en verre (4) chaud avec des courbes de tolérance prédéterminées,
> g. de génération d'un signal d'alarme si ladite courbe de différences relatives dépasse lesdites courbes de tolérance en au moins un point.

11. Système selon la revendication 9 ou 10, **caractérisé en ce que** ladite unité de processeur (10) est en outre programmée pour effectuer l'étape suivante afin de commander le processus de formage automatiquement :

h. d'envoi de ladite courbe de différences relatives de chaque récipient en verre (4) chaud à ladite unité de commande (7).

12. Système selon l'une quelconque des revendications 7 à 11, **caractérisé en ce que** ladite unité de mesure (9) est sensible aux longueurs d'onde entre 0,7 et 3,0 microns.

13. Système selon la revendication 12, **caractérisé en ce que** ladite unité de mesure (9) comprend une caméra infrarouge à ondes courtes (SWIR).

FIG. 1

Fig. 2a

Fig. 2b

Fig. 2c

Fig. 2d

Fig. 2e

Fig. 2f

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5583337 A
- US 20070102628 A
- WO 2004011935 A
- EP 1525469 B1

### Non-patent literature cited in the description

- Automated inspection and container monitoring at the hot end. **DR. JOHN CHAN.** international Glass Review, Contract Communications, London. 01 January 1997, 109-111
- **ROBERTO MICHELETTI.** Automatic visual inspection for glass production. *ISMCR. Proceedings of the International Symposium on Measurement and Control in Robotics,* 01 January 1998, 127-131